# EUROPÄISCHE PATENTANMELDUNG

(11) **EP 1 240 894 A2**
(43) Veröffentlichungstag der Anmeldung: **18.09.2002**
(21) Anmeldenummer: 02003206.6
(22) Anmeldetag: 19.02.2002
(51) Int. Cl.: A61K 7/42

(54) **Verwendung von Lichtschutzmittelkombinationen in kosmetischen und pharmazeutischen Zubereitungen**

(30) Priorität: 15.03.2001 DE 10113058
(71) Anmelder: BASF AKTIENGESELLSCHAFT, 67056 Ludwigshafen (DE)
(72) Erfinder: Heidenfelder, Thomas, Dr., 67125 Dannstadt (DE); Tiefensee, Kirstin, Dr., 67098 Bad Dürkheim (DE); Wünsch, Thomas, Dr., 67346 Speyer (DE)

(57) **Zusammenfassung**

Verwendung von Lichtschutzmittelkombinationen, enthaltend eine
A) im wesentlichen im W-A-Bereich absorbierende Verbindung und
B) weitere im UV-A-Bereich, im UV-B-Bereich und über beide Bereiche absorbierende Verbindungen,
wobei der im UV-A-Bereich absorbierende Bestandteil aus
A) wirksamen Mengen mindestens einer der Verbindungen der Formel I, 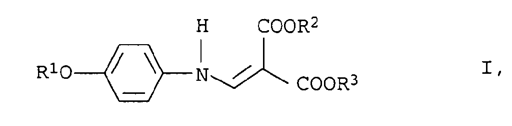 in der die Substituenten R¹ bis R³ unabhängig voneinander C₁-C₈-Alkyl bedeuten, besteht
als photostabile UV-Filter in kosmetischen und pharmazeutischen Zubereitungen zum Schutz der menschlichen Haut und menschlicher Haare gegen Sonnenstrahlen.

## Beschreibung

Die Erfindung betrifft die Verwendung von Lichtschutzmittelkombinationen, die als wesentlichen Bestandteil die im UV-A-Bereich absorbierenden 2-(4-Alkoxy-anilinomethylen)-malonsäuredialkylester enthalten und mindestens ein weiteres Lichtschutzmittel, das im UV-A-, im UV-B-Bereich oder in beiden Bereichen absorbiert, ausgewählt aus einer im einzelnen im folgenden definierten Gruppe als photostabile UV-Filter-Kombination in kosmetischen und pharmazeutischen Zubereitungen zum Schutz der menschlichen Epidermis oder menschlichen Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hohe spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, die Lichtschutzmittel erfüllen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 und EP-A-0 416 837 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und je nach Substituent in Öl oder Wasser löslich sind.

Es wurde nun gefunden, daß diese Aufgabe vorteilhaft durch bestimmte Lichtschutzmittelkombinationen gelöst werden kann.

Demgemäß wurde diese Aufgabe erfindungsgemäß gelöst durch die Verwendung von Lichtschutzmittelkombinationen, enthaltend eine
A) im wesentlichen im UV-A-Bereich absorbierende Verbindung und
B) weitere im UV-A-Bereich, im UV-B-Bereich und über beide Bereiche absorbierende Verbindungen,
wobei der im UV-A-Bereich absorbierende Bestandteil aus
A) wirksamen Mengen mindestens einer der Verbindungen der Formel I, 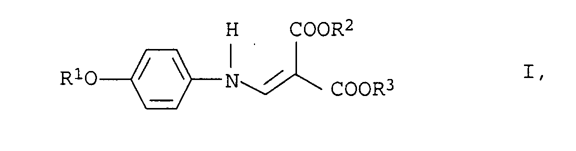 in der die Substituenten R¹ bis R³ unabhängig voneinander C₁-C₈-Alkyl bedeuten, besteht
   und der Bestandteil
B) wirksame Mengen einer oder mehrerer Verbindungen enthält, ausgewählt aus der Gruppe bestehend aus
Ba) Hydroxybenzophenone der allgemeinen Formel II 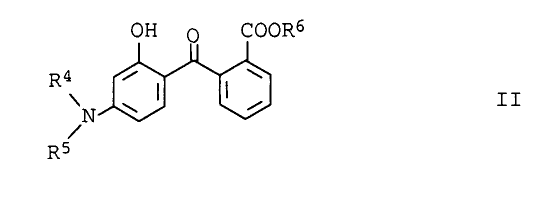 in der die Substituenten unabhängig voneinander folgende Bedeutung haben:
   - R⁴ und R⁵: Wasserstoff, C₁-C₁₂-Alkyl, C₃-C₁₀-Cycloalkyl, wobei die Substituenten R⁴ und R⁵ gemeinsam mit dem Stickstoffatom, an dem sie gebunden sind, einen 5- oder 6-Ring bilden können und
   - R⁶: Wasserstoff, C₁-C₁₂-Alkyl, C₃-C₁₀-Cycloalkyl;
Bb) Diarylbutadiene der Formel III 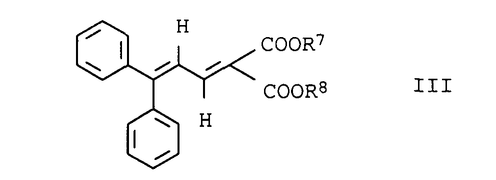 in der die Substituenten R⁷ und R⁸ unabhängig voneinander Wasserstoff, C₁-C₁₂-Alkyl oder C₃-C₁₀-Cycloalkyl bedeuten können,
Bc) der Verbindung der Formel IV 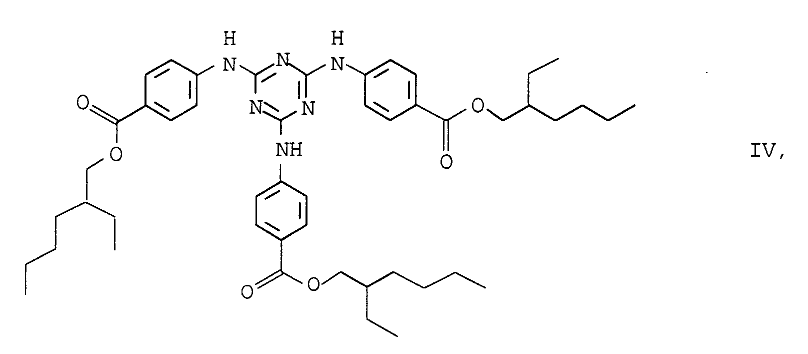
Bd) der Verbindung der Formel V 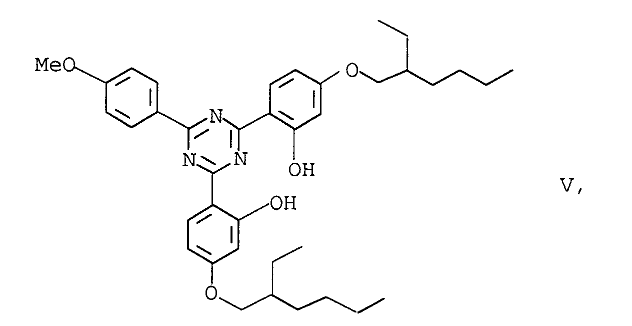
Be) der Verbindung der Formel VI 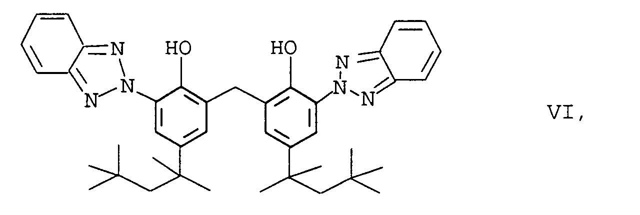
Bf) der Verbindung der Formel VII 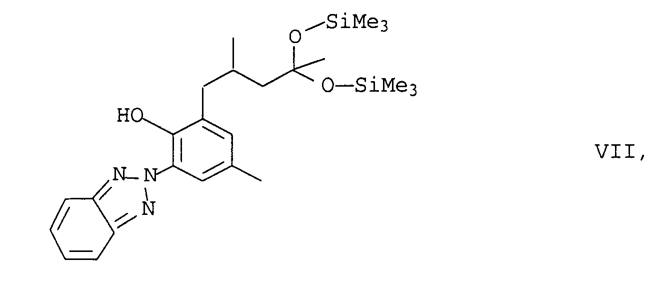
Bg) einem Organosiloxanbenzalmalonat der Formel VIIIa 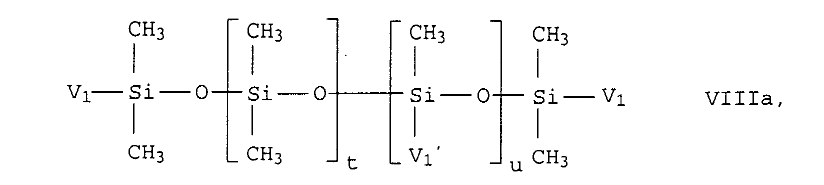 in der
   - V₁': die Gruppe 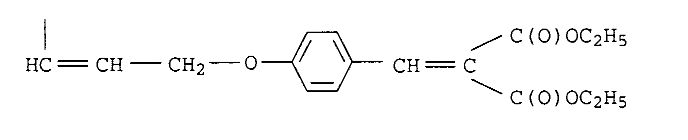
   - V₁: eine Methylgruppe oder V₁' bedeutet oder der Formel VIIIb 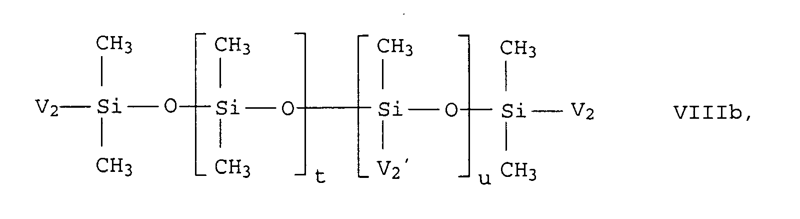
   in der V₂' die Gruppe der Struktur 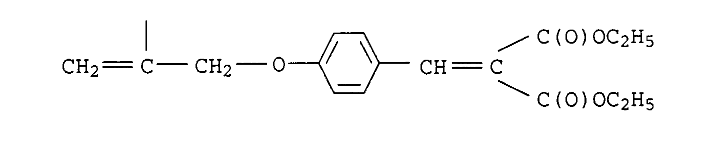
   - V₂: eine Methylgruppe oder V₂' bedeutet
   oder Mischungen aus Verbindungen der Formeln VIIIa und VIIIb,
   wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
Bh) der Verbindung der Formel IX 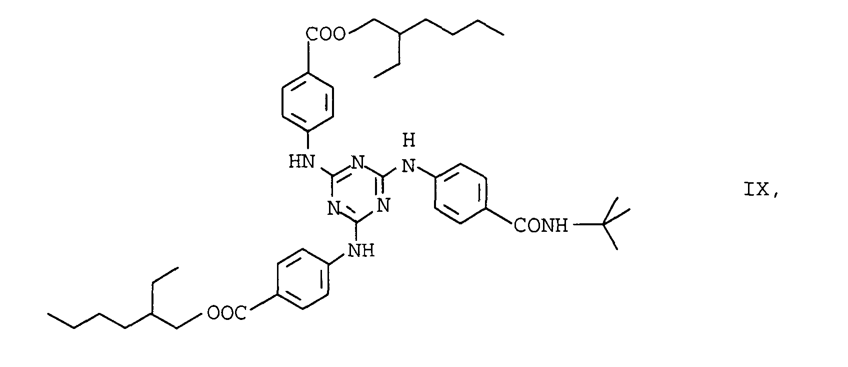
Bi) der Verbindung der Formel X 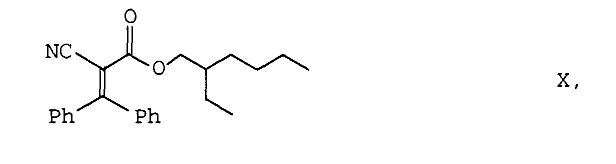
Bj) der Verbindung der Formel XI 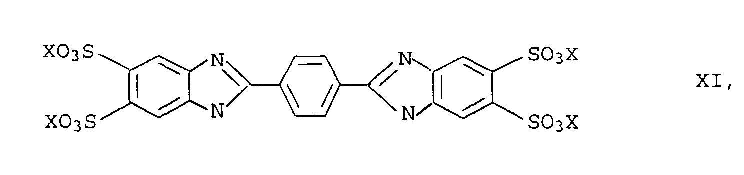 in der X Wasserstoff, Natrium, Kalium, Ammonium oder Triethanolammonium bedeutet,
Bk) der Verbindung der Formel XII 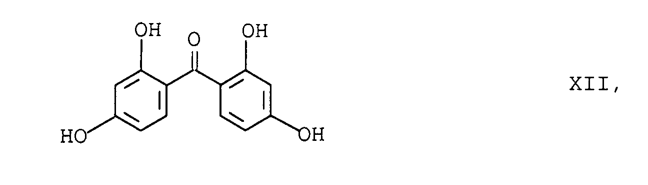 als photostabile UV-Filter in kosmetischen und pharmazeutischen Zubereitungen zum Schutz der menschlichen Haut oder menschlicher Haare gegen UV-Strahlen, insbesondere gegen natürliche Sonnenstrahlen, gegebenenfalls zusammen mit weiteren an sich für kosmetische und pharmazeutische Zubereitungen bekannten, im UV-Bereich absorbierenden Verbindungen.
   Als Alkylreste R¹ bis R³ in Verbindung I seien verzweigte oder unverzweigte C₁-C₈-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl oder n-Octyl genannt. Bevorzugte Reste für R¹ bis R³ sind Methyl, Ethyl, n-Propyl und 1-Methylethyl.
   Als Alkylreste R⁴, R⁵ und R⁶ der Verbindung II seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder 2-Ethylhexyl genannt.
   Als besonders bevorzugte Alkylreste seien für R⁴, R⁵ und R⁶ Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.
   Als Alkylreste R⁷ und R⁸ der Verbindung III seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder 2-Ethylhexyl genannt.
   Als besonders bevorzugte Alkylreste seien für R⁷ und R⁸ Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.
   Als Cycloalkylreste seien für R⁴ bis R⁸ der Formeln II und III bevorzugt verzweigte oder unverzweigte C₃-C₁₀-Cycloalkylketten wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl, Cyclooctyl, Cyclononyl oder Cyclodecyl genannt.
   Als besonders bevorzugte Cycloalkylreste für R⁴ bis R⁸ seien C₃-C₆-Cycloalkylreste, ganz besonders bevorzugt Cyclopropyl, Cyclopentyl und Cyclohexyl genannt.
   Die Lichtschutzmittelkombination aus den Verbindungen (A) und (B) kann allein oder in Verbindung mit weiteren im UV-Bereich absorbierenden Verbindungen angewandt werden, doch sollen mindestens wirksame Mengen der Verbindungen (A) bzw. (B) in den Lichtschutzpräparaten enthalten sein. Unter wirksamen Mengen der Verbindungen (A) und der Verbindungen (B) werden im allgemeinen jeweils mindestens 0,1 Gew.-%, jeweils bezogen auf die kosmetische Zubereitung, verstanden.
   In den erfindungsgemäßen Lichtschutzmittelkombinationen überwiegt in der Regel die Menge der im UV-B-Bereich absorbierenden Verbindungen. Demgemäß beträgt der Gehalt der im UV-A-Bereich absorbierenden Verbindung (A) im allgemeinen 5 bis 50 Gew.-%, vorzugsweise 10 bis 25 Gew.-%, jeweils bezogen auf Lichtschutzmittelkombination aus (A) und (B).
   Die Lichtschutzmittelkombinationen zeichnen sich durch ihre hohe Photostabilität, die mit den Lichtschutzmittelkombinationen hergestellten Zubereitungen durch ihr angenehmes Hautgefühl aus. Die mit den Kombinationen erzielten Lichtschutzfaktoren sind deutlich höher als die additiven Werte der einzelnen UV-Filter. Auch die UVA-Schutzleistung der erfindungsgemäßen Lichtschutzmittelkombinationen - hinsichtlich der kritischen Wellenlänge, dem PPD-Wert (persistent pigment darkening), dem IPD-Wert (immediate pigment darkening) sowie dem UVA/UVB-Verhältnis - ist höher als die, die durch die bekannten, dem Stand der Technik gemäßen UV-Filter erzielbar ist.
   Die Verbindung der Formel I ist in US 6,069,170 als Bestandteil kosmetischer Zubereitungen beschrieben. Darüber hinaus ist in Spalte 3 der genannten Patentschrift, Zeilen 5 bis 27 erwähnt, daß es möglich ist, die Verbindungsgruppe, zu der die Verbindung der Formel I gehört, zusammen mit bekannten UltraviolettAbsorbern zu verwenden. Es konnte jedoch aus diesen Angaben nicht geschlossen werden, daß mit bestimmten, ausgewählten UV-Absorbern besonders vorteilhafte Kombinationen erhalten werden können.
   Die Verbindungen (B) sind sämtlich bekannt und werden im folgenden näher charakterisiert:
   Ba) Die Verbindung der Formel II ist aus EP-A-1 046 391 bekannt.
   Bb) Die Verbindung der Formel III ist aus EP-A-0 916335 bekannt.
   Bc) Die Verbindung der Formel IV hat die CAS Nr. 88122-99-0 und wird unter dem Markennamen Uvinul® T150 (BASF) vertrieben.
   Bd) Die Verbindung der Formel V hat die CAS Nr. 187393-00-6.
   Be) Die Verbindung der Formel VI hat die CAS Nr. 103597-45-1 und wird unter dem Markennamen Tinosorb® M (CIBA) vertrieben.
   Bf) Die Verbindung der Formel VII hat die CAS Nr. 155633-54-8 und ist unter dem Markennamen Mexoryl® XL (CHIMEX) bekannt.
   Bg) Die Verbindungen bzw. Gemische von Verbindungen der Formel VIII sind aus EP-A 0920859 bekannt. Von den Verbindungen der Formeln VIIIa und/oder VIIIb kommen vor allem die mit den CAS Nummern 208391-15-5, 208391-15-5D, 177955-90-7, 177955-90-7D und 177995-90-7DP in Betracht.
   Bh) Die Verbindung der Formel IX hat die CAS Nr. 154702-15-5 und wird unter dem Markennamen Uvasorb® HEB (BV Sigma) vertrieben.
   Bi) Die Verbindung der Formel X hat die CAS Nr. 6197-30-4 und wird unter dem Markennamen Uvinul® N539 (BASF) vertrieben.
   Bj) Die Verbindung der Formel XI hat die CAS Nr. 180898-37-7 (Haarmann & Reimer).
   Bk) Die Verbindung der Formel XII hat die CAS Nr. 131-55-5 und wird unter dem Markennamen Uvinul® D50 (BASF) vertrieben.

In den erfindungsgemäß zu verwendenden Lichtschutzmittelkombinationen können nicht nur einzelne der Verbindungen (Ba) bis (Bk), sondern auch Mischungen aus mehreren dieser Verbindungen enthalten sein.

Gegenstand der vorliegenden Erfindung sind weiterhin kosmetische und pharmazeutische Zubereitungen, die 0,1 bis 40 Gew.-%, vorzugsweise 1 bis 25 Gew.-%, bezogen auf die gesamte Menge der kosmetischen und pharmazeutischen Zubereitung einer Lichtschutzmittelkombination aufweisen, enthaltend eine
A) im wesentlichen im UV-A-Bereich absorbierende Verbindung und
B) weitere im UV-A-Bereich, im UV-B-Bereich und über beide Bereiche absorbierende Verbindungen aufweisen,
   wobei der im UV-A-Bereich absorbierende Bestandteil aus
   A) wirksamen Mengen der Verbindung der Formel I 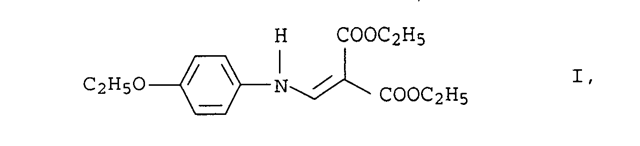 besteht
      und der Bestandteil
   B) wirksame Mengen einer oder mehrerer Verbindungen enthält, ausgewählt aus der Gruppe bestehend aus
   Ba) Hydroxybenzophenon der Formel IIa, 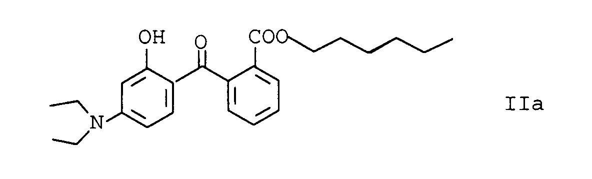
   Bb) Diarylbutadien der Formel IIIa 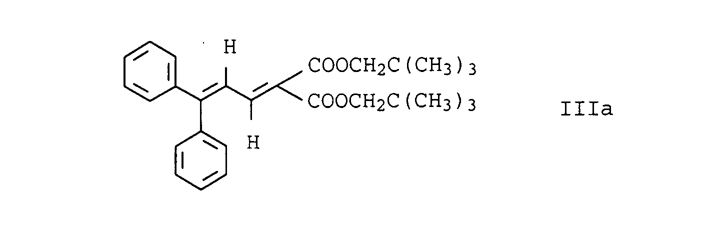
   Bc) der Verbindung der Formel IV 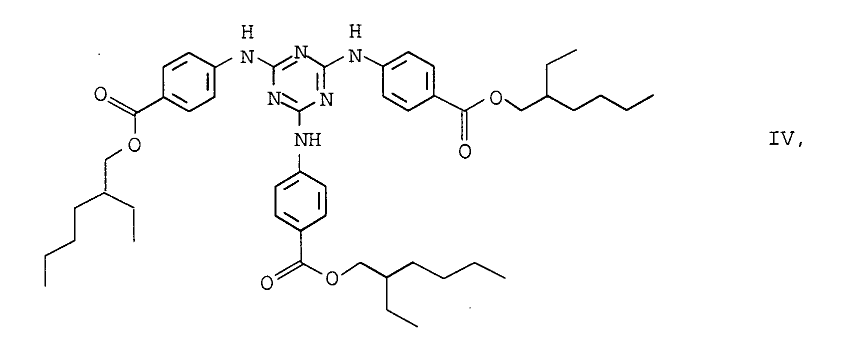
   Bd) der Verbindung der Formel V 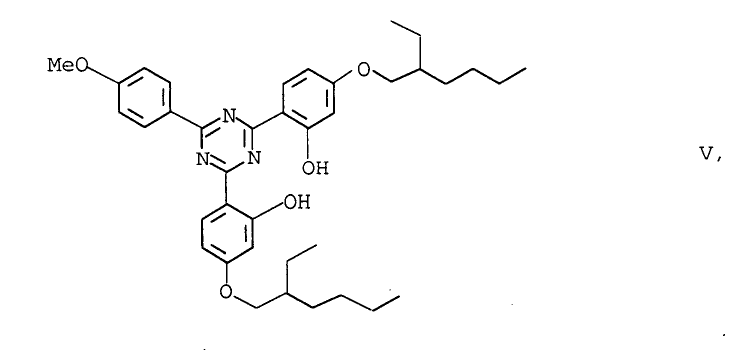
   Be) der Verbindung der Formel VI 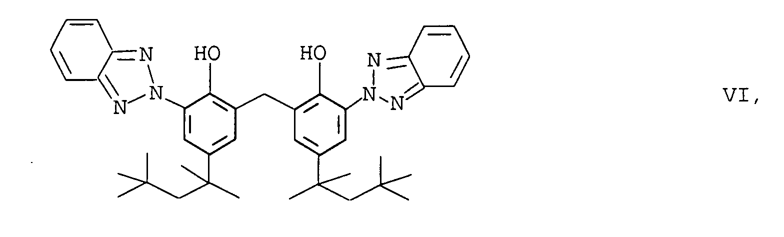
   Bf) der Verbindung der Formel VII 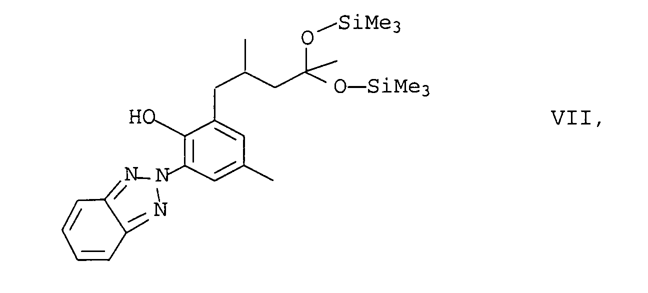
   Bg) einem Organosiloxanbenzalmalonat der Formel VIIIa 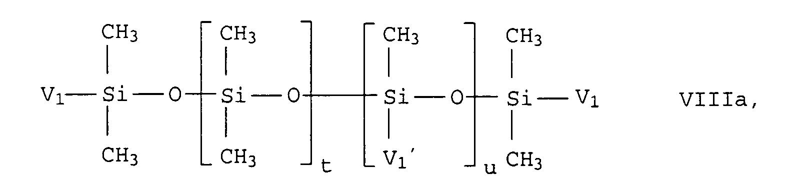 in der
      - V₁': die Gruppe 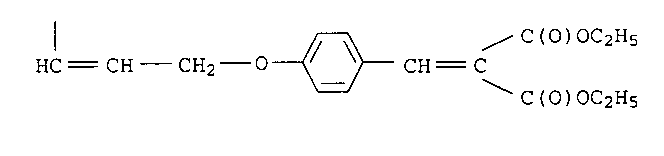
      - V₁: eine Methylgruppe oder V₁' bedeutet oder der Formel VIIIb 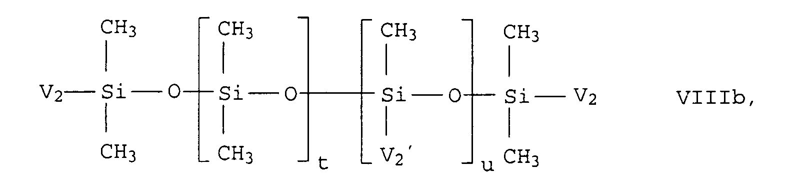 in der V₂' die Gruppe der Struktur 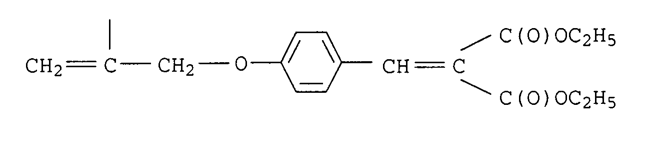
      - V₂: eine Methylgruppe oder V₂' bedeutet
      oder Mischungen aus Verbindungen der Formeln VIIIa und VIIIb,
      wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
   Bh) der Verbindung der Formel IX 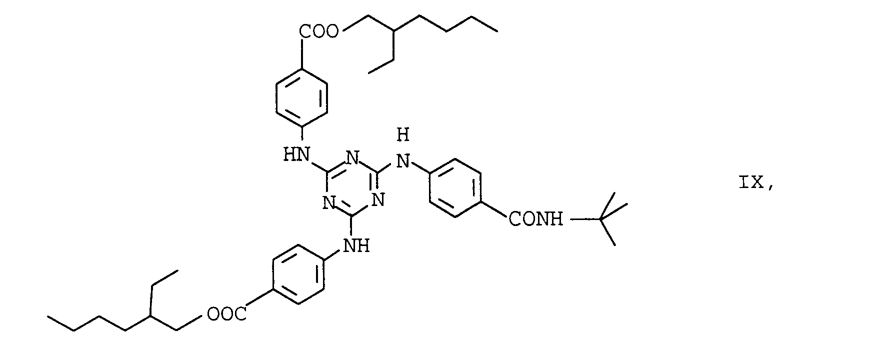
   Bi) der Verbindung der Formel X 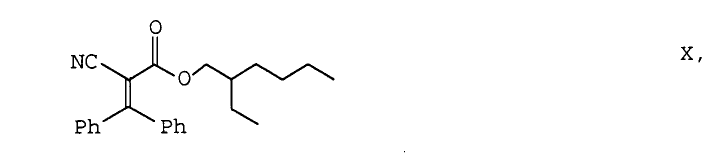
   Bj) der Verbindung der Formel XI 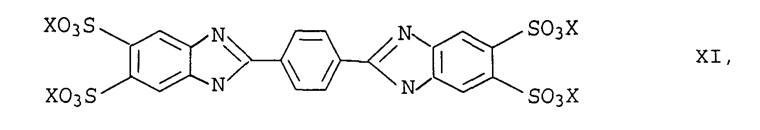 in der X Wasserstoff, Natrium, Kalium, Ammonium oder Triethanolammonium bedeutet,
   Bk) der Verbindung der Formel XII, 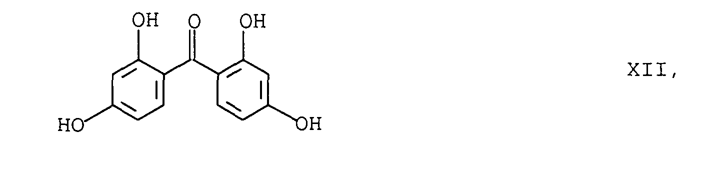 gegebenenfalls zusammen mit weiteren an sich für kosmetische und pharmazeutische Zubereitungen bekannten, im UV-A- und UV-B-Bereich absorbierenden Verbindungen, wobei die UV-A absorbierenden Verbindungen in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit der in dispergierter Form vorliegenden Verbindung der Formel I in Betracht, wobei die Emulsionen durch Phaseninversionstechnologie, gemäß DE-A-197 26 121 erhältlich sind. Dabei wählt man die Partikelgröße der Verbindung der Formel I so, daß sie 3 µm oder weniger, vorzugsweise 1 µm oder weniger der mittleren Teilchengröße beträgt.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminiumund/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. ß-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasserin-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Als UV-Filtersubstanzen, die zusätzlich mit den erfindungsgemäß zu verwendenden Lichtschutzmittelkombinationen angewandt werden können, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-ethylhexylester | 5466-77-3 |
| 11 | 2-Hydroxy-4-methoxy-benzophenon-5-sulfonsäure (Sulisobenzonum) und das Natriumsalz | 4065-45-6 |
| 12 | 3-(4'-Sulfo)benzyliden-bornan-2-on und Salze | 58030-58-6 |
| 13 | 3-Benzylidenbornan-2-on | 16087-24-8 |
| 14 | 1-(4'-Isopropylphenyl)-3-phenylpropan-1,3-dion | 63260-25-9 |
| 15 | 4-Isopropylbenzylsalicylat | 94134-93-7 |
| 16 | 3-Imidazol-4-yl-acrylsäure und ihr Ethylester | 104-98-3 |
| 17 | 2-Cyano-3,3-diphenylacrylsäureethylester | 5232-99-5 |
| 18 | Menthyl-o-aminobenzoate oder: 5-Methyl-2-(1-methylethyl)-2-aminobenzoate | 134-09-8 |
| 19 | Glyceryl p-aminobenzoat oder: 4-Aminobenzoesäure-1-glyceryl-ester | 136-44-7 |
| 20 | 2,2'-Dihydroxy-4-methoxybenzophenon (Dioxybenzone) | 131-53-3 |
| 21 | 2-Hydroxy-4-methoxy-4-methylbenzophenon (Mexonon) | 1641-17-4 |
| 22 | Triethanolamin Salicylat | 2174-16-5 |
| 23 | Dimethoxyphenylglyoxalsäure oder: 3,4-dimethoxy-phenyl-glyoxal-saures Natrium | 4732-70-1 |
| 24 | 3-(4'Sulfo)benzyliden-bornan-2-on und seine Salze | 56039-58-8 |
| 25 | 4-tert.-Butyl-4'-methoxy-dibenzoylmethan | 70356-09-1 |
| 26 | 3-(4-Methylbenzyliden)-campher | 36861-47-9 |

Die erfindungsgemäßen kosmetischen und dermatologischen Zubereitungen können vorteilhafterweise außerdem weitere anorganische Pigmente auf Basis von Metalloxiden und/oder anderen in Wasser schwerlöslichen oder unlöslichen Metallverbindungen, insbesondere der Oxide des Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäß zu verwendenden Lichtschutzmittelkombinationen in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Lichtschutzmittelkombinationen sind gut in kosmetischen Ölen löslich und lassen sich leicht in kosmetische Formulierungen einarbeiten. Die mit den neuen Lichtschutzmittelkombinationen hergestellten Emulsionen zeichnen sich besonders durch ihre hohe Stabilität, die Lichtschutzmittelkombinationen selber durch ihre hohe Photostabilität aus, und die mit den Lichtschutzmittelkombinationen hergestellten Zubereitungen durch ihr angenehmes Hautgefühl aus.

Die UV-Filterwirkung der erfindungsgemäß zu verwendenden Lichtschutzmittelkombinationen kann auch zur Stabilisierung von Wirk- und Hilfsstoffen in kosmetischen und pharmazeutischen Formulierungen ausgenutzt werden.

In den folgenden Beispielen wird die Verwendung der neuen Lichtschutzmittelkombinationen näher erläutert.

### Beispiele

### Beispiel 1 - Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 2 - Zusammensetzung für die Lippenpflege Massengehalt (Gew.-%)

ad 100 Eucerinum anhydricum
10,00 Glycerin
Filterkombination bestehend aus
5,00 (I)
8,00 (II)
10,00 TiO₂
5,00 ZnO₂
4,00 Castoröl
4,00 Pentaerythrityl Stearat/caprat/Caprylat Adipat
3,00 Glyceryl Stearat SE
2,00 Bienenwachs
2,00 Microkristallines Wachs
2,00 Quaternium-18 Bentonit
1,50 PEG-45/Dodecyl Glycol Copolymer

### Beispiel 3 - Zusammensetzung für die Lippenpflege Massengehalt (Gew.-%)

ad 100 Eucerinum anhydricum
10,00 Glycerin
Filterkombination bestehend aus
1,00 (I)
8,00 (II)
4,00 (V)
10,00 TiO₂
5,00 ZnO₂
4,00 Castoröl
4,00 Pentaerythrityl Stearat/caprat/Caprylat Adipat
3,00 Glyceryl Stearat SE
2,00 Bienenwachs
2,00 Microkristallines Wachs
2,00 Quaternium-18 Bentonit
1,50 PEG-45/Dodecyl Glycol Copolymer

### Beispiel 4 - Zusammensetzung für Sunblocker mit Mikropigmenten Massengehalt (Gew.-%)

ad 100 Wasser
6,00 PEG-7-Hydrogenated Castor Öl
Filterkombination bestehend aus
2,00 (I)
3,00 (V)
10,00 (II)
3,00 (X)
6,00 TiO₂
5,00 Mineral Öl
5,00 Isoamyl p-Methoxycinnamat
5,00 Propylen Glycol
3,00 Jojoba Öl
2,00 PEG-45/Dodecyl Glycol Copolymer
1,00 Dimethicon
0,50 PEG-40-Hydrogenated Castor Öl
0,50 Tocopheryl Acetat
0,50 Phenoxyethanol
0,20 EDTA

### Beispiel 5 - Zusammensetzung für Sunblocker mit Mikropigmenten Massengehalt (Gew.-%)

ad 100 Wasser
6,00 PEG-7-Hydrogenated Castor Öl
Filterkombination bestehend aus
2,00 (I)
3,00 (IV)
10,00 (II)
3,00 (X)
6,00 TiO₂
5,00 Mineral Öl
5,00 Isoamyl p-Methoxycinnamat
5,00 Propylen Glycol
3,00 Jojoba Öl
2,00 PEG-45/Dodecyl Glycol Copolymer
1,00 Dimethicon
0,50 PEG-40-Hydrogenated Castor Öl
0,50 Tocopheryl Acetat
0,50 Phenoxyethanol
0,20 EDTA

### Beispiel 6 - Fettfreies Gel Massengehalt (Gew.-%)

ad 100 Wasser
Filterkombination bestehend aus
2,00 (I)
3,00 (VI)
8,00 (II)
1,00 (X)
7,00 TiO₂
5,00 Glycerin
5,00 PEG-25 PABA
0,40 Acrylate C₁₀-C₃₀ Alkyl Acrylat Crosspolymer
0,30 Imidazolidinyl Urea
0,25 Hydroxyethyl Cellulose
0,25 Sodium Methylparaben
0,20 Disodium EDTA
0,15 Fragrance
0,15 Sodium Propylparaben
0,10 Sodium Hydroxid

### Beispiel 7 - Fettfreies Gel Massengehalt (Gew.-%)

ad 100 Wasser
Filterkombination bestehend aus
1,00 (I)
4,00 (VI)
8,00 (II)
1,00 (X)
7,00 TiO₂
5,00 Glycerin
5,00 PEG-25 PABA
0,40 Acrylate C₁₀-C₃₀ Alkyl Acrylat Crosspolymer
0,30 Imidazolidinyl Urea
0,25 Hydroxyethyl Cellulose
0,25 Sodium Methylparaben
0,20 Disodium EDTA
0,15 Fragrance
0,15 Sodium Propylparaben
0,10 Sodium Hydroxid

### Beispiel 8 - Sonnencreme Massengehalt (Gew.-%)

ad 100 Wasser
Filterkombination bestehend aus
2,00 (I)
3,00 (XIV)
8,00 (II)
1,00 (X)
8,00 TiO₂
5,00 ZnO₂
6,00 PEG-7-Hydrogenated Castor Öl
6,00 Mineral Öl
5,00 Isopropyl Palmitat
0,30 Imidazolidinyl Urea
3,00 Jojoba Öl
2,00 PEG-45/Dodecyl Glycol Copolymer
0,60 Magnesium Stearat
0,50 Tocopheryl Acetat
0,25 Methylparaben
0,20 Disodium EDTA
0,15 Propylparaben

### Beispiel 9 - Sonnencreme Massengehalt (Gew.-%)

ad 100 Wasser
Filterkombination bestehend aus
3,00 (I)
3,00 (XI)
8,00 (II)
8,00 TiO₂
5,00 ZnO₂
6,00 PEG-7-Hydrogenated Castor Öl
6,00 Mineral Öl
5,00 Isopropyl Palmitat
0,30 Imidazolidinyl Urea
3,00 Jojoba Öl
2,00 PEG-45/Dodecyl Glycol Copolymer
0,60 Magnesium Stearat
0,50 Tocopheryl Acetat
0,25 Methylparaben
0,20 Disodium EDTA
0,15 Propylparaben

### Beispiel 10 - Sonnencreme wasserfest Massengehalt (Gew.-%)

ad 100 Wasser
Filterkombination bestehend aus
1,00 (I)
4,00 (IX)
8,00 (II)
2,00 (X)
3,00 TiO₂
5,00 PEG-7-Hydrogenated Castor Öl
5,00 Propylene Glycol
4,00 Isopropyl Palmitat
4,00 Caprylic/Capric Triglycerid
4,00 Glycerin
3,00 Jojoba Öl
1,50 PEG-45/Dodecyl Glycol Copolymer
1,50 Dimethicon
0,70 Magnesium Sulfat
0,50 Magnesium Stearat
0,15 Fragrance

### Beispiel 11 - Sonnencreme wasserfest Massengehalt (Gew.-%)

ad 100 Wasser
Filterkombination bestehend aus
2,00 (I)
5,00 (XII)
2,00 TiO₂
5,00 PEG-7-Hydrogenated Castor Öl
5,00 Propylene Glycol
4,00 Isopropyl Palmitat
4,00 Caprylic/Capric Triglycerid
4,00 Glycerin
3,00 Jojoba Öl
1,50 PEG-45/Dodecyl Glycol Copolymer
1,50 Dimethicon
0,70 Magnesium Sulfat
0,50 Magnesium Stearat
0,15 Fragrance

### Beispiel 12 - Sonnenmilch Massengehalt (Gew.-%)

ad 100 Wasser
10,00 Mineral Öl
6,00 PEG-7-Hydrogenated Castor Öl
5,00 Isopropyl Palmitat
Filterkombination bestehend aus
3,00 (I)
5,50 (VIII)
3,00 Caprylic/Capric Triglycerid
3,00 Jojoba Öl
2,00 PEG-45/Dodecyl Glycol Copolymer
0,70 Magnesium Sulfat
0,60 Magnesium Stearat
0,50 Tocopheryl Acetat
3,00 Glycerin
0,25 Methylparaben
0,15 Propylparaben
0,05 Tocopherol

### Beispiel 13 - Sonnenmilch Massengehalt (Gew.-%)

ad 100 Wasser
10,00 Mineral Öl
6,00 PEG-7-Hydrogenated Castor Öl
5,00 Isopropyl Palmitat
Filterkombination bestehend aus
1,00 (I)
4,00 (XIV)
3,50 (II)
3,00 Caprylic/Capric Triglycerid
3,00 Jojoba Öl
2,00 PEG-45/Dodecyl Glycol Copolymer
0,70 Magnesium Sulfat
0,60 Magnesium Stearat
0,50 Tocopheryl Acetat
3,00 Glycerin
0,25 Methylparaben
0,15 Propylparaben
0,05 Tocopherol

## Patentansprüche

1. Verwendung von Lichtschutzmittelkombinationen, enthaltend eine
A) im wesentlichen im UV-A-Bereich absorbierende Verbindung und
B) weitere im UV-A-Bereich, im UV-B-Bereich und über beide Bereiche absorbierende Verbindungen,
wobei der im UV-A-Bereich absorbierende Bestandteil aus
A) wirksamen Mengen mindestens einer der Verbindungen der Formel I besteht, 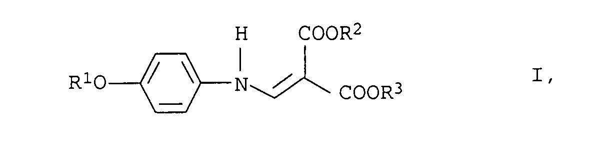 in der die Substituenten R¹ bis R³ unabhängig voneinander C₁-C₈-Alkyl bedeuten
und der Bestandteil
B) wirksame Mengen einer oder mehrerer Verbindungen enthält, ausgewählt aus der Gruppe bestehend aus
Ba) Hydroxybenzophenone der allgemeinen Formel II 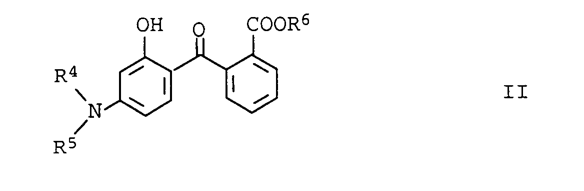 in der die Substituenten unabhängig voneinander folgende Bedeutung haben:
R⁴ und R⁵ Wasserstoff, C₁-C₁₂-Alkyl, C₃-C₁₀-Cycloalkyl, wobei die Substituenten R⁴ und R⁵ gemeinsam mit dem Stickstoffatom, an dem sie gebunden sind, einen 5- oder 6-Ring bilden können und
R⁶ Wasserstoff, C₁-C₁₂-Alkyl, C₃-C₁₀-Cycloalkyl;
Bb) Diarylbutadiene der Formel III 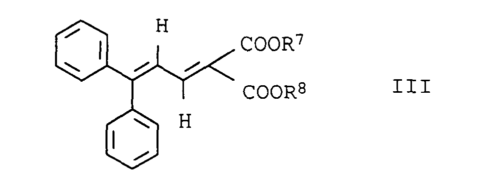 in der die Substituenten R⁷ und R⁸ unabhängig voneinander Wasserstoff, C₁-C₁₂-Alkyl oder C₃-C₁₀-Cycloalkyl bedeuten können,
Bc) der Verbindung der Formel IV 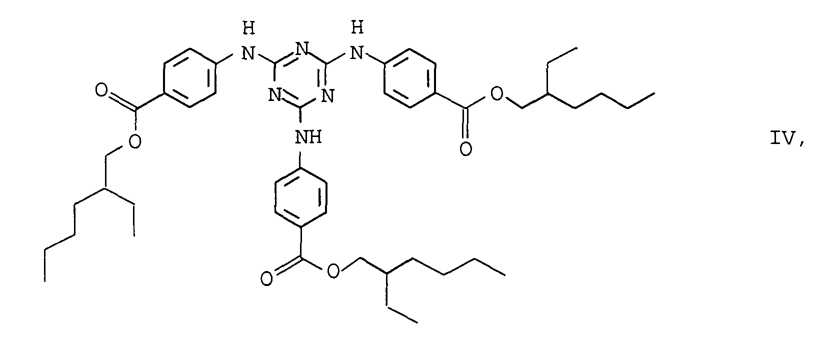
Bd) der Verbindung der Formel V 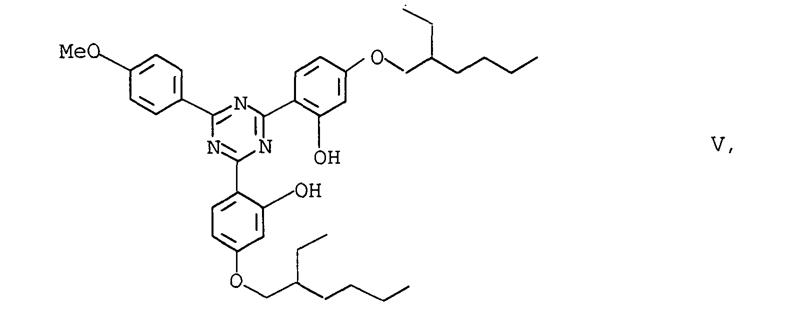
Be) der Verbindung der Formel VI 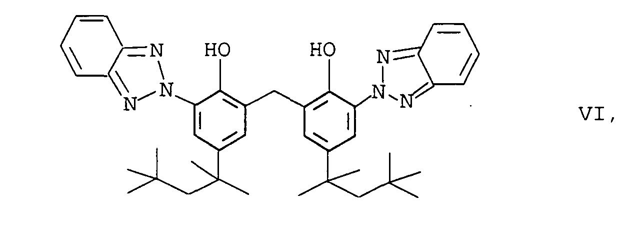
Bf) der Verbindung der Formel VII 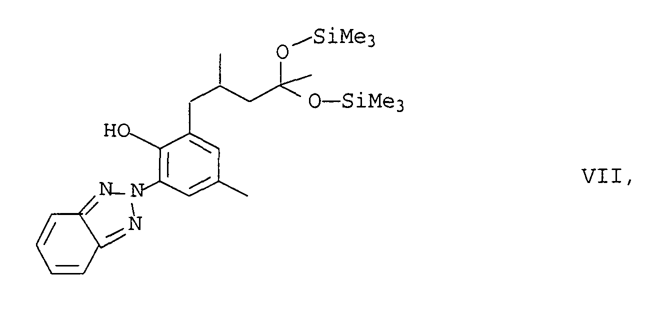
Bg) einem Organosiloxanbenzalmalonat der Formel VIIIa 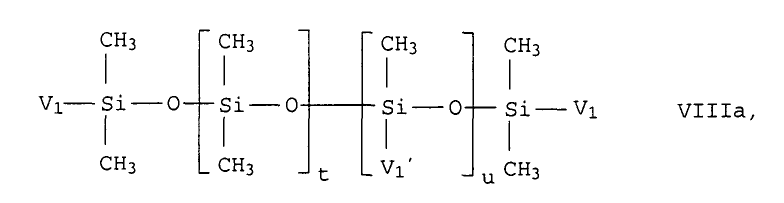 in der
V₁' die Gruppe 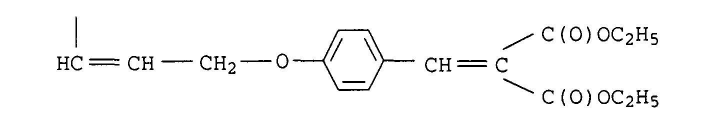
V₁ eine Methylgruppe oder V₁' bedeutet oder der Formel VIIIb 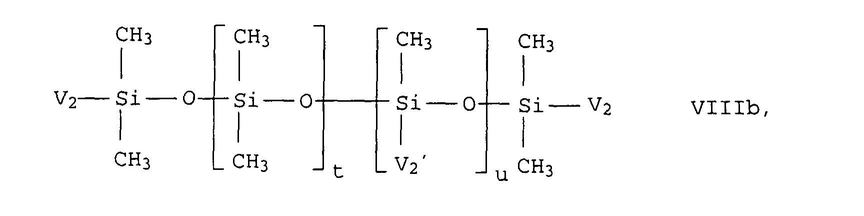 in der V₂' die Gruppe der Struktur 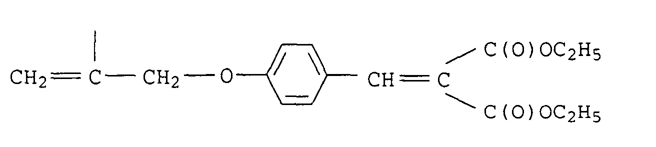
V₂ eine Methylgruppe oder V₂' bedeutet
oder Mischungen aus Verbindungen der Formeln VIIIa und VIIIb,
wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
Bh) der Verbindung der Formel IX 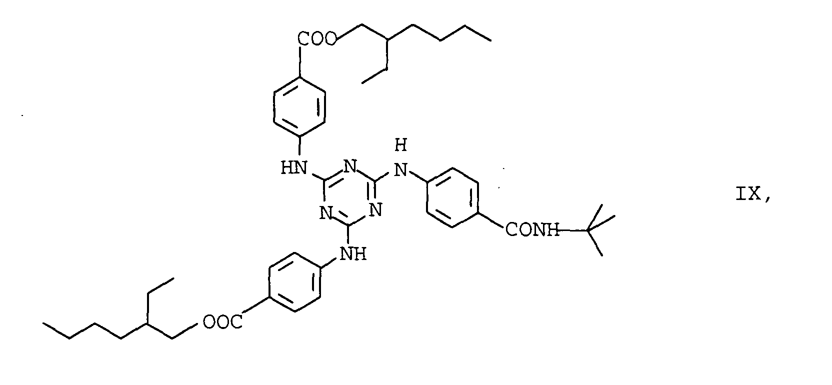
Bi) der Verbindung der Formel X 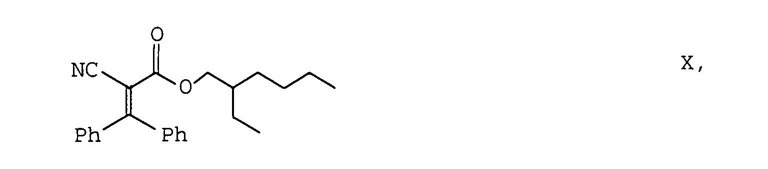
Bj) der Verbindung der Formel XI 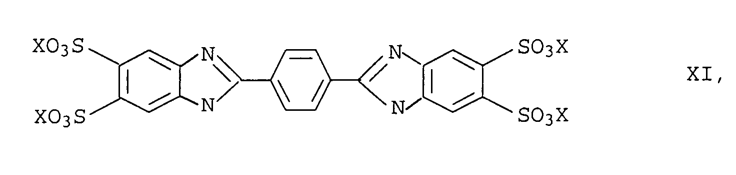 in der X Wasserstoff, Natrium, Kalium, Ammonium oder Triethanolammonium bedeutet,
Bk) der Verbindung der Formel XII 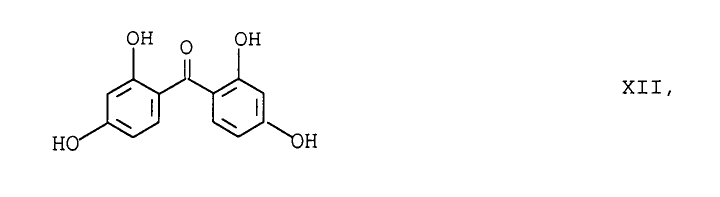 als photostabile UV-Filter in kosmetischen und pharmazeutischen Zubereitungen zum Schutz der menschlichen Haut oder menschlicher Haare gegen Sonnenstrahlen, gegebenenfalls zusammen mit weiteren an sich für kosmetische und pharmazeutische Zubereitungen bekannten, im UV-Bereich absorbierenden Verbindungen.

2. Verwendung von Lichtschutzmittelkombinationen gemäß Anspruch 1, **dadurch gekennzeichnet, daß** sie den Bestandteil A) der Formel I in Mengen von mindestens 5 Gew.-%, bezogen auf die Lichtschutzmittelkombination, enthalten.

3. Lichtschutzmittelkombinationen enthaltende kosmetische und pharmazeutische Zubereitungen zum Schutz der menschlichen Epidermis oder der menschlichen Haare gegen UV-Licht im Bereich von 280 bis 400 nm, **dadurch gekennzeichnet, daß** sie in einem kosmetisch und pharmazeutisch geeigneten Träger als photostabile UV-Filter wirksame Mengen von Lichtschutzmittelkombinationen enthalten, die
A) eine im wesentlichen im UV-A-Bereich absorbierende Verbindung und
B) weitere im UV-A-Bereich, im UV-B-Bereich und über beide Bereiche absorbierende Verbindungen aufweisen,
wobei der im UV-A-Bereich absorbierende Bestandteil aus
A) wirksamen Mengen der Verbindung der Formel 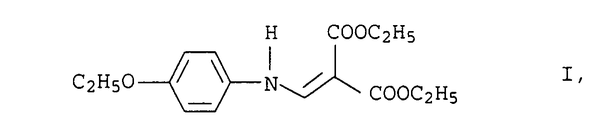 besteht
und der Bestandteil
B) wirksame Mengen einer oder mehrerer Verbindungen enthält, ausgewählt aus der Gruppe bestehend aus
Ba) Hydroxybenzophenon der Formel IIa, 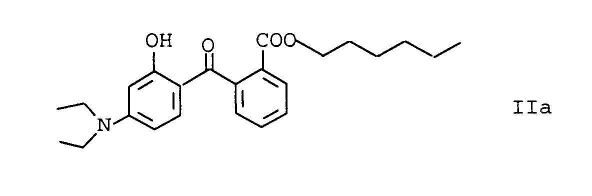
Bb) Diarylbutadien der Formel IIIa, 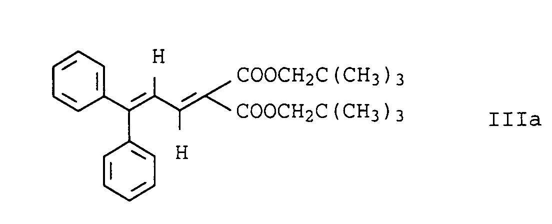
Bc) der Verbindung der Formel IV 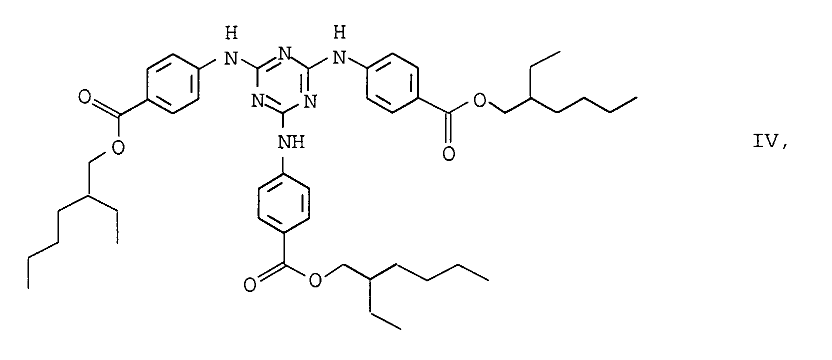
Bd) der Verbindung der Formel V 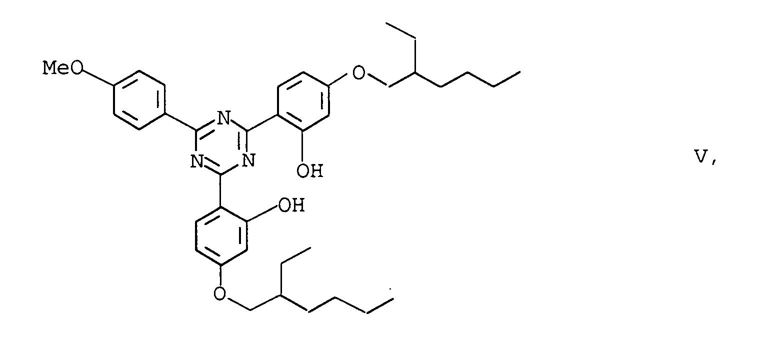
Be) der Verbindung der Formel VI 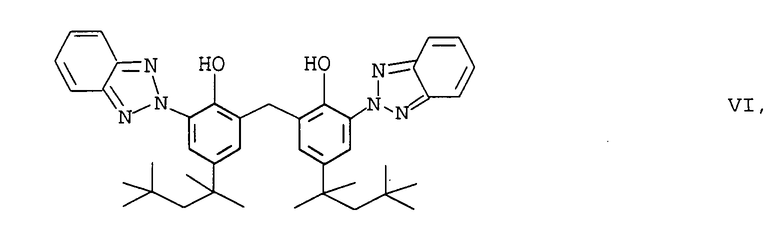
Bf) der Verbindung der Formel VII 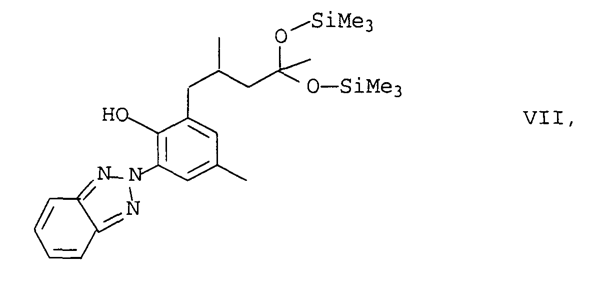
Bg) einem Organosiloxanbenzalmalonat der Formel VIIIa 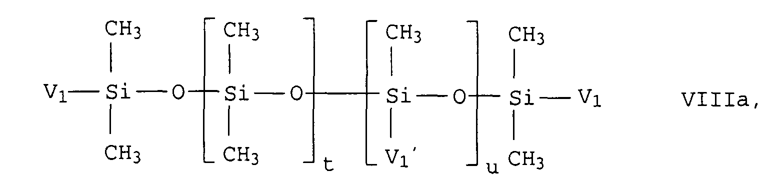 in der
V₁' die Gruppe 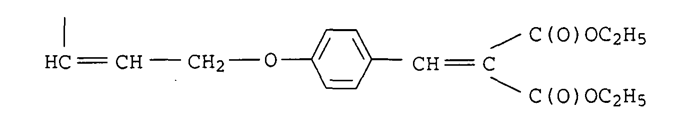
V₁ eine Methylgruppe oder V₁' bedeutet oder der Formel VIIIb 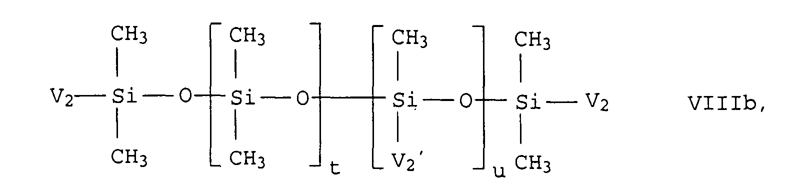
in der V₂' die Gruppe der Struktur 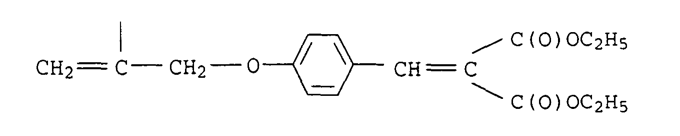
V₂ eine Methylgruppe oder V₂' bedeutet
oder Mischungen aus Verbindungen der Formeln VIIIa und VIIIb, wobei t für einen Wert bis 100 und u für einen Wert bis 20 steht mit der Maßgabe, daß u = 0 ist, wenn V₁ = V₁' und/oder V₂ = V'₂ ist und u einen Wert von 1 bis 20 bedeutet, wenn V₁ = CH₃ und/oder V₂ = CH₃ ist,
Bh) der Verbindung der Formel IX 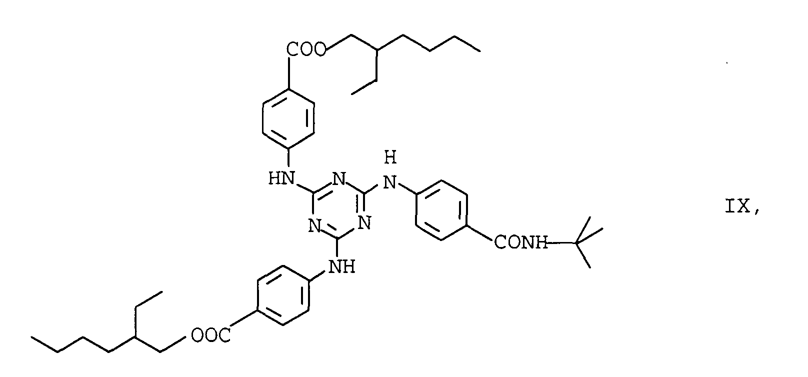
Bi) der Verbindung der Formel X 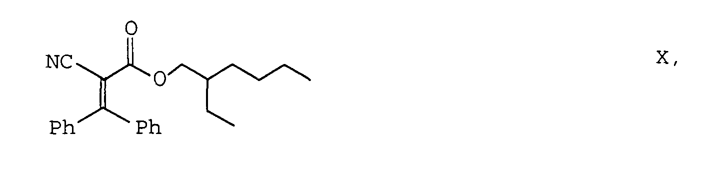
Bj) der Verbindung der Formel XI 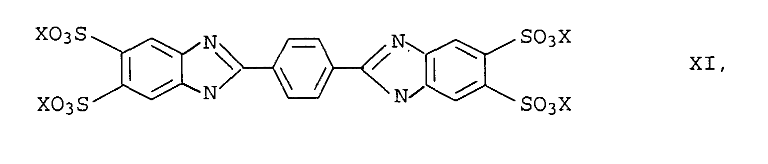 in der X Wasserstoff, Natrium, Kalium, Ammonium oder Triethanolammonium bedeutet,
Bk) der Verbindung der Formel XII 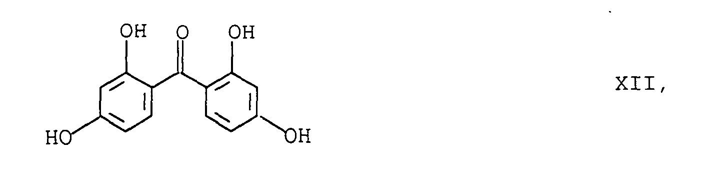 gegebenenfalls zusammen mit weiteren an sich für kosmetische und pharmazeutische Zubereitungen bekannten, im UV-Bereich absorbierenden Verbindungen.

4. Lichtschutzmittelkombinationen enthaltende kosmetische und pharmazeutische Zubereitungen gemäß Anspruch 3, **dadurch gekennzeichnet, daß** die Lichtschutzmittelkombinationen den Bestandteil der Formel I in Mengen von mindestens 5 Gew.-%, bezogen auf die Lichtschutzmittelkombination, enthalten.
